# EUROPEAN PATENT APPLICATION

(11) **EP 4 840 539 A1**
(43) Date of publication of application: **18.11.2026**
(21) Application number: 25849630.6
(22) Date of filing: 22.09.2025
(51) Int. Cl.: B01J 20/20, A61M 16/01, C01B 32/30

(54) **ANESTHETIC GAS ADSORBENT, ANESTHETIC GAS ADSORBING FILTER, INHALATION ANESTHESIA SYSTEM, AND ANESTHETIC GAS REMOVAL METHOD**

(30) Priority: 28.03.2025 JP 2025056675; 28.03.2025 JP 2025056676
(71) Applicant: Osaka Gas Chemicals Co., Ltd., Osaka-shi, Osaka 550-0023 (JP)
(72) Inventor: IMANISHI, Masachiyo, Osaka-shi, Osaka 550-0023 (JP); NAKATANI, Tomoaki, Osaka-shi, Osaka 550-0023 (JP); CHISHIRO, Takefumi, Osaka-shi, Osaka 550-0023 (JP); YUASA, Hiroto, Osaka-shi, Osaka 550-0023 (JP)
(74) Representative: SSM Sandmair
(86) International application number: PCT/JP2025/033418
(87) International publication number: WO 2026/203456

(57) **Abstract**

The anesthetic gas adsorbent has activated carbon. The "pore volume of pores having pore radii of 0.6 nm or less" of the activated carbon calculated by the MP method is 0.41 mL/g or more. The ratio of the "pore volume of pores having pore radii of 0.4 nm or less" of the activated carbon calculated by the MP method to the "pore volume of pores having pore radii of 0.5 nm or less" of the activated carbon calculated by the MP method is 50% or more.

## Description

### TECHNICAL FIELD

The present invention relates to an anesthetic gas adsorbent, an anesthetic gas adsorption filter, an inhalation anesthesia system, and a method for removing anesthetic gas.

### BACKGROUND ART

There has been conventionally known a method in which waste anesthetic gas containing a volatile anesthetic is brought into contact with an adsorbent to adsorb and remove the volatile anesthetic in the waste anesthetic gas (for example, see Patent Document 1).

### CITATION LIST

### PATENT DOCUMENT

PATENT DOCUMENT 1: Japanese Unexamined Patent Publication No. 2002-172171

### SUMMARY OF THE INVENTION

### TECHNICAL PROBLEM

For adsorbents such as described in Patent Document 1, there has been a demand for further improvement in adsorption performance for volatile anesthetics.

The present invention provides an anesthetic gas adsorbent, an anesthetic gas adsorption filter, an inhalation anesthesia system, and a method for removing anesthetic gas, each capable of improving the adsorption performance for anesthetic gas contained in exhaust gas.

### SOLUTION TO THE PROBLEM

An aspect [1] of the present invention includes an anesthetic gas adsorbent including activated carbon, a "pore volume of pores having pore radii of 0.6 nm or less" of the activated carbon calculated by MP method being 0.41 mL/g or more, a ratio of a "pore volume of pores having pore radii of 0.4 nm or less" of the activated carbon calculated by the MP method to a "pore volume of pores having pore radii of 0.5 nm or less" of the activated carbon calculated by the MP method being 50% or more.

An aspect [2] of the present invention includes the anesthetic gas adsorbent of the above aspect [1], wherein a packing density of the activated carbon excluding an ignition residue is more than 0.38 g/mL and less than 0.50 g/mL.

An aspect [3] of the present invention includes the anesthetic gas adsorbent of the above aspect [1] or [2], wherein the "pore volume of pores having pore radii of 0.6 nm or less" of the activated carbon calculated by the MP method is 1.70 mL/g or less.

An aspect [4] of the present invention includes the anesthetic gas adsorbent of any one of the above aspects [1] to [3], wherein a specific surface area of the activated carbon is 950 m²/g or more and 2000 m²/g or less, and a butane activity of the activated carbon is 24.4% or more and 40.0% or less.

An aspect [5] of the present invention includes an anesthetic gas adsorption filter including the anesthetic gas adsorbent of any one of the above aspects [1] to [4].

An aspect [6] of the present invention includes the anesthetic gas adsorption filter of the above aspect [5], further including: a container having an inlet port that is located at an end portion of the container on a first side in a first direction and that allows exhaust gas containing anesthetic gas to flow into the container, and an outlet port that is located at another end portion of the container on a second side in the first direction and that allows the exhaust gas, from which the anesthetic gas has been removed, to flow out of the container; the anesthetic gas adsorbent disposed between the inlet port and the outlet port in the first direction; and a spacer disposed between the inlet port and the anesthetic gas adsorbent in the first direction.

An aspect [7] of the present invention includes the anesthetic gas adsorption filter of the above aspect [6], wherein the spacer is a protrusion protruding from an inner surface of the container on the first side in the first direction toward the anesthetic gas adsorbent.

An aspect [8] of the present invention includes an inhalation anesthesia system including: a vaporizer configured to vaporize a volatile anesthetic agent to generate anesthetic gas and to supply the generated anesthetic gas to inspiratory gas from a ventilator; and an anesthetic gas adsorption filter connected to an exhaust line of the ventilator and including the anesthetic gas adsorbent of any one of the above aspects [1] to [4].

An aspect [9] of the present invention includes the inhalation anesthesia system of the above aspect [8], further including the ventilator.

An aspect [10] of the present invention includes a method for removing anesthetic gas, the method including passing exhaust gas containing anesthetic gas through the anesthetic gas adsorbent of any one of the above aspects [1] to [4], thereby removing the anesthetic gas from the exhaust gas.

### ADVANTAGES OF THE INVENTION

The anesthetic gas adsorbent of the present invention includes the activated carbon, the "pore volume of pores having pore radii of 0.6 nm or less" is 0.41 mL/g or more, and the ratio of the "pore volume of pores having pore radii of 0.4 nm or less" to the "pore volume of pores having pore radii of 0.5 nm or less" is 50% or more.

It is therefore possible to improve the adsorption performance for anesthetic gas contained in exhaust gas.

The anesthetic gas adsorption filter, the inhalation anesthesia system, and the method for removing anesthetic gas according to embodiments of the present invention use the anesthetic gas adsorbent described above.

Thus, anesthetic gas contained in exhaust gas can be efficiently removed.

### BRIEF DESCRIPTION OF THE DRAWINGS

[FIG. 1] FIG. 1 is an explanatory diagram for explaining an activation step of a method for producing an anesthetic gas adsorbent.
[FIG. 2] FIG. 2 is a sectional view of an anesthetic gas adsorption filter according to an embodiment of the present invention.
[FIG. 3] FIG. 3 is a block diagram for explaining the configuration of an inhalation anesthesia system according to an embodiment of the present invention.
[FIG. 4] FIG. 4 is a sectional view of the anesthetic gas adsorption filter of Variation (2).
[FIG. 5] FIG. 5 is a sectional view of the anesthetic gas adsorption filter of Variation (3).
[FIG. 6] FIG. 6A is a perspective view of a lid shown in FIG. 5. FIG. 6B is a plan view of the lid shown in FIG. 6A, as viewed from the inside.
[FIG. 7] FIG. 7A is a sectional view of the anesthetic gas adsorption filter of Variation (4). FIG. 7B is a view of the lid shown in FIG. 7A, as viewed from a second side in a first direction.
[FIG. 8] FIG. 8 is an explanatory diagram for explaining the lid of Variation (5).
[FIG. 9] FIG. 9 is an explanatory diagram for explaining the lid of Variation (6).
[FIG. 10] FIG. 10 is an explanatory diagram for explaining the lid of Variation (7).

### DESCRIPTION OF EMBODIMENTS

### 1. Anesthetic Gas Adsorbent

The following describes an anesthetic gas adsorbent as an embodiment of the present invention.

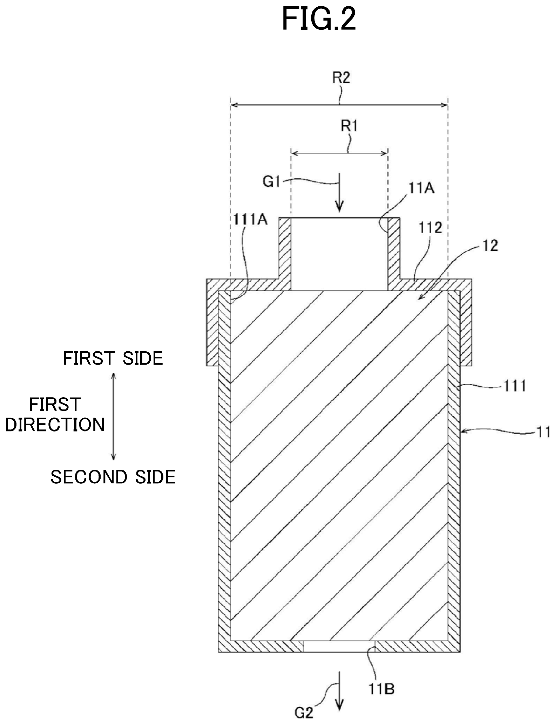

The anesthetic gas adsorbent is used in an anesthetic gas adsorption filter 10 (see FIG. 2) of an inhalation anesthesia system 100. The anesthetic gas adsorption filter 10 and the inhalation anesthesia system 100 will be described later. The anesthetic gas adsorbent has activated carbon. The anesthetic gas adsorbent may consist solely of activated carbon. Activated carbon is capable of adsorbing anesthetic gas. Examples of the activated carbon include wood-based activated carbon, coconut shell-based activated carbon, and coal-based activated carbon. The activated carbon can be preferably coconut shell-based activated carbon or coal-based activated carbon, more preferably coconut shell-based activated carbon.

### (1) Details of Activated Carbon

The pore volume of the activated carbon calculated by the MP method is, for example, 0.43 mL/g to 0.70 mL/g, preferably 0.48 mL/g to 0.66 mL/g.

The "pore volume of pores having pore radii of 0.6 nm or less" of the activated carbon calculated by the MP method is 0.41 mL/g or more, preferably 0.43 mL/g or more, more preferably 0.44 mL/g or more. When the "pore volume of pores having pore radii of 0.6 nm or less" of the activated carbon calculated by the MP method is equal to or greater than the above lower limit, the adsorption performance for anesthetic gas contained in exhaust gas ("adsorption performance") can be improved.

It should be noted that twice the "pore radius" of the activated carbon determined by the MP method equals the "pore diameter" of the activated carbon determined by the MP method. Accordingly, for example, the "pore volume of pores having pore radii of 0.6 nm or less" of the activated carbon calculated by the MP method is equal to the "pore volume of pores having pore diameters of 1.2 nm or less" of the activated carbon calculated by the MP method. Thus, in this description, the "pore volume of pores having pore radii of 0.6 nm or less" of the activated carbon calculated by the MP method is replaceable with the "pore volume of pores having pore diameters of 1.2 nm or less" of the activated carbon calculated by the MP method, the "pore volume of pores having pore radii of 0.5 nm or less" of the activated carbon calculated by the MP method is replaceable with the "pore volume of pores having pore diameters of 1.0 nm or less" of the activated carbon calculated by the MP method, and the "pore volume of pores having pore radii of 0.4 nm or less" of the activated carbon calculated by the MP method is replaceable with the "pore volume of pores having pore diameters of 0.8 nm or less" of the activated carbon calculated by the MP method.

The upper limit of the "pore volume of pores having pore radii of 0.6 nm or less" of the activated carbon calculated by the MP method is not particularly limited. The "pore volume of pores having pore radii of 0.6 nm or less" of the activated carbon calculated by the MP method is, for example, 1.70 mL/g or less, preferably 1.20 mL/g or less, more preferably 0.70 mL/g or less. When the "pore volume of pores having pore radii of 0.6 nm or less" of the activated carbon calculated by the MP method is equal to or less than the above upper limit, the anesthetic gas adsorbent is less likely to become excessively bulky. This can mitigate an increase in the size of the anesthetic gas adsorption filter.

The range of the "pore volume of pores having pore radii of 0.6 nm or less" of the activated carbon calculated by the MP method is, for example, 0.41 mL/g to 1.70 mL/g, preferably 0.43 mL/g to 1.20 mL/g, more preferably 0.44 mL/g to 0.70 mL/g.

The "pore volume of pores having pore radii of 0.6 nm or less" is measured by the method described in Examples below.

The ratio (V_{0.4}/V_{0.5}) of the "pore volume V_{0.4} of pores having pore radii of 0.4 nm or less" of the activated carbon calculated by the MP method to the "pore volume V_{0.5} of pores having pore radii of 0.5 nm or less" of the activated carbon calculated by the MP method is 50% or more. To be more specific, the ratio (V_{0.4}/V_{0.5}) is a percentage of the "pore volume V_{0.4} of pores having pore radii of 0.4 nm or less" to the "pore volume V_{0.5} of pores having pore radii of 0.5 nm or less," and is calculated by the following formula. Formula: 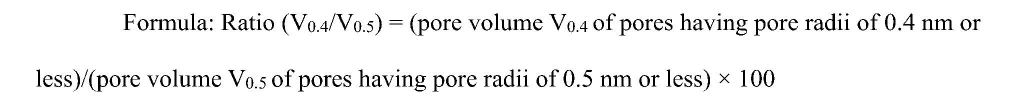 Ratio (V0.4/V0.5) = (pore volume V0.4 of pores having pore radii of 0.4 nm or less)/(pore volume V0.5 of pores having pore radii of 0.5 nm or less) × 100

The "pore volume V_{0.5} of pores having pore radii of 0.5 nm or less" and the "pore volume V_{0.4} of pores having pore radii of 0.4 nm or less" are measured by the method described in the Examples below.

When the ratio (V_{0.4}/V_{0.5}) is equal to or greater than the above lower limit, the adsorption performance for anesthetic gas contained in exhaust gas ("adsorption performance") can be further improved.

The ratio (V_{0.4}/V_{0.5}) is, for example, 99% or less, preferably 91% or less, more preferably 89% or less, even more preferably 87% or less. When the ratio (V_{0.4}/V_{0.5}) is equal to or less than the above upper limit, a certain proportion of pores having pore radii between 0.4 nm and 0.5 nm, inclusive, can be ensured, contributing to securing migration paths for anesthetic gas molecules into pores having pore radii of 0.4 nm or less. As a result, a decrease in adsorption performance can be mitigated.

The ratio (V_{0.4}/V_{0.5}) is, for example, 50% to 99%, preferably 50% to 91%, more preferably 50% to 89%, even more preferably 50% to 87%.

The bulk density of the activated carbon is, for example, 0.40 g/mL to 0.50 g/mL.

The bulk density of the activated carbon is measured by the method described in the Examples below.

The activated carbon contains a carbon component and an ignition residue. The activated carbon does not have to contain components other than the carbon component and the ignition residue. In other words, the activated carbon may consist solely of the carbon component and the ignition residue.

The carbon component of the activated carbon is capable of adsorbing anesthetic gas. The packing density of the carbon component is calculated by subtracting the ignition residue from the bulk density of the activated carbon. Specifically, the packing density of the carbon component is calculated by the following formula. Formula: 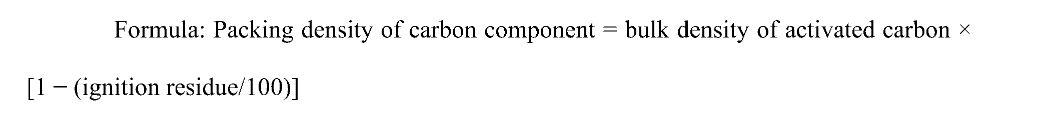 Packing density of carbon component = bulk density of activated carbon × [1 - (ignition residue/100)]

The packing density of the activated carbon excluding the ignition residue (i.e., the packing density of the carbon component) is more than 0.38 g/mL and less than 0.50 g/mL. The packing density of the carbon component is preferably 0.39 g/mL or more. When the packing density of the carbon component is more than the above lower limit, the volume of the carbon component can be ensured with respect to the volume of the activated carbon. When the packing density of the carbon component is less than the above upper limit, the pore volume of the activated carbon can be ensured. Thus, when the packing density of the carbon component is within the above range, the adsorption performance can be improved.

The ignition residue of activated carbon does not contribute to adsorption of anesthetic gas. The ignition residue of the activated carbon is, for example, 1.0 mass% to 15.0 mass%, preferably 1.0 mass% to 5.0 mass%.

The ignition residue of the activated carbon is measured by the method described in the Examples below.

The specific surface area of the activated carbon is, for example, 950 m²/g or more, preferably 1050 m²/g or more. When the specific surface area of the activated carbon is equal to or greater than the above lower limit, sufficient adsorption performance can be ensured. The upper limit of the specific surface area of the activated carbon is not particularly limited. The specific surface area of the activated carbon may be 2000 m²/g or less or may be 1700 m²/g or less.

The range of the specific surface area of the activated carbon may be 950 m²/g to 2000 m²/g, or 1050 m²/g to 1700 m²/g.

The specific surface area is measured by the method described in the Examples below.

The butane activity of the activated carbon is, for example, 24.4% or more, preferably 25.0% or more. When the butane activity of the activated carbon is equal to or greater than the above lower limit, sufficient adsorption performance can be ensured. The upper limit of the butane activity of the activated carbon is not particularly limited. The butane activity of the activated carbon may be 40.0% or less or may be 35.0% or less.

The range of the butane activity of the activated carbon may be 24.4% to 40.0%, or 25.0% to 35.0%.

### (2) Details of Anesthetic Gas

The anesthetic gas adsorbent is preferably used for adsorbing an anesthetic gas represented by the following general formula (1).

### General formula (1):

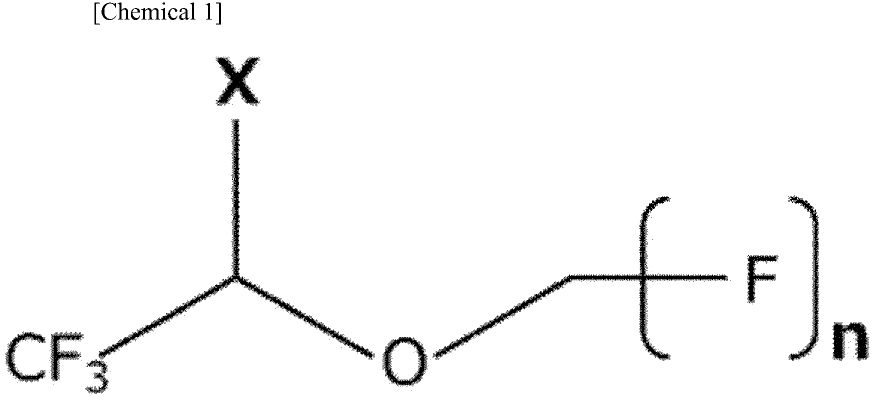

(X is a hydrogen atom, a fluorine atom, a chlorine atom, a bromine atom, an iodine atom, or a perfluoromethyl group; and n is 0 or an integer of 1 to 3.)

Examples of the anesthetic gas represented by the general formula (1) above include isoflurane, desflurane, and sevoflurane.

Specifically, when X is a chlorine atom and n is 2 in the above general formula (1), the anesthetic gas is isoflurane (molecular weight: 184.5, boiling point: 48.5°C, vapor pressure at 20°C: 31.7 kPa).

When X is a perfluoromethyl group and n is 1 in the above general formula (1), the anesthetic gas is sevoflurane (molecular weight: 200.6, boiling point: 58.5°C, vapor pressure at 20°C: 20.9 kPa).

When X is a fluorine atom and n is 2 in the above general formula (1), the anesthetic gas is desflurane (molecular weight: 168.4, boiling point: 23.5°C, vapor pressure at 20°C: 88.5 kPa).

### 2. Method for Producing Activated Carbon

The following describes a method for producing the anesthetic gas adsorbent.

In the production of the anesthetic gas adsorbent, first, activated carbon is produced. Examples of method for producing activated carbon include a pyrolysis method, an activation method, a coating method, and a vapor deposition method. The method for producing activated carbon can be preferably activation method.

The activation method includes an activation step. The activation method may include a carbonization step and a removal step.

### (1) Carbonization Step

In the carbonization step, the raw material of activated carbon is carbonized. When the raw material of activated carbon is coal, the method for producing activated carbon does not have to include the carbonization step.

Examples of the raw material of activated carbon include plant-based raw materials, fossil-based raw materials, synthetic resins, synthetic rubber, synthetic wood, and synthetic pulp. Examples of the plant-based raw materials include wood, wood flour, fruit shells, seeds, byproducts of pulp production, bagasse, and waste molasses. Examples of the fruit shells include coconut shells. Examples of the seeds include palm kernels, Japanese apricot (Prunus mume) seeds, and peach seeds. Examples of the fossil-based raw materials include coal, anthracite, petroleum distillation residue, petroleum pitch, coke, and coal tar. Examples of the synthetic resins include phenolic resin, vinyl chloride resin, vinyl acetate resin, melamine resin, urea resin, resorcinol resin, celluloid, epoxy resin, polyurethane resin, polyester resin, acrylic resin, and polyamide resin. Examples of the synthetic rubber include polybutylene, polybutadiene, and polychloroprene. One of the raw materials may be used alone, or two or more of the raw materials may be used in combination.

The raw material of activated carbon can be preferably any of the plant-based raw materials or the fossil-based raw materials, more preferably any of the coconut shells or the coal.

Heating conditions in the carbonization step are not particularly limited. In the carbonization step, for example, the raw material is heated at, for example, 300°C to 900°C, preferably 400°C to 800°C in an oxygen-free environment.

Heating time in the carbonization step is not particularly limited. The heating time is, for example, 15 minutes to 20 hours, preferably 30 minutes to 10 hours.

The carbonization step may be performed under reduced pressure and may be performed under a nitrogen atmosphere. The carbonization step is performed using, for example, a rotary kiln.

In the carbonization step, the raw material may be pulverized or molded before the raw material is carbonized.

When the raw material is pulverized, the particle size (D50, i.e., the 50% particle diameter in the cumulative volume-based distribution) of the raw material is, for example, 1 µm to 150 µm.

In the carbonization step, the raw material that has been carbonized (i.e., carbonized raw material) may be pulverized. When the carbonized raw material is pulverized, the particle size (D50, i.e., the 50% particle diameter in the cumulative volume-based distribution) of the carbonized raw material is, for example, 1 µm to 150 µm.

In the carbonization step, after the raw material (or the carbonized raw material) is pulverized, an additive may be added, as needed, to the pulverized raw material (or the pulverized carbonized raw material), the mixture may be kneaded, and the obtained kneaded mixture may be molded. In this way, the activated carbon described above can be easily produced.

Examples of such additives include water, coal tar, dewatered tar, hard pitch, coal-tar pitch, and petroleum pitch. One of the additives may be used alone, or two or more of the additives may be used in combination.

The additive is blended, for example, in the range of 1.0 part by mass to 100.0 parts by mass, preferably in the range of 1.0 parts by mass to 50.0 parts by mass, with respect to 100 parts by mass of the raw material (or the carbonized raw material).

When the kneaded mixture is molded into a cylindrical shape, the diameter of the molded article is preferably 0.1 mm to 4.0 mm or less. The aspect ratio (height/diameter) of the molded article is, for example, 1 to 10.

### (2) Removal Step

The removal step is performed after the carbonization step and before the activation step. In the removal step, causing substances causing an ignition residue (e.g., soil and sand) are removed from the carbonized raw material.

Examples of the method for removing the causing substances from the carbonized raw material include wet or dry soil-and-sand separation and heavy-media separation using a liquid having a high specific gravity.

### (3) Activation Step

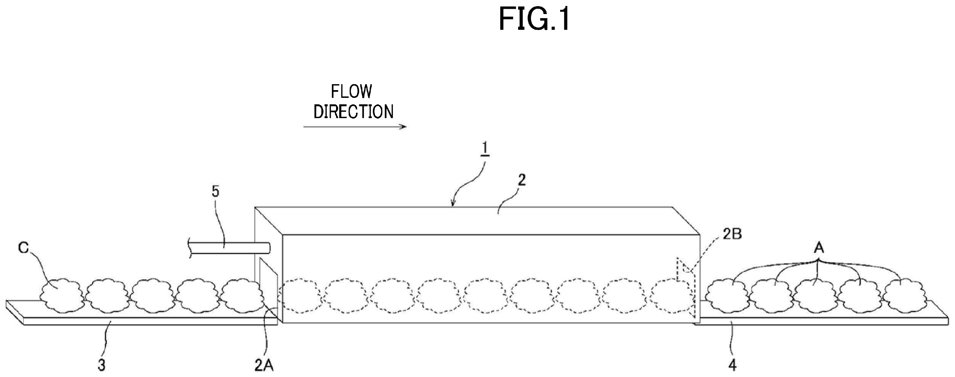

As shown in FIG. 1, in the activation step, a carbonized raw material C, from which the causing substances have been removed, is activated using an activation system 1.

The activation system 1 includes an activation furnace 2, a supply device 3, a conveyance device 4, and an activation gas supply device 5. The activation system 1 is a continuous system. Specifically, in the activation system 1, while the carbonized raw material C is continuously supplied to the activation furnace 2, activated carbon A activated in the activation furnace 2 is continuously discharged from the activation furnace 2.

### (3-1) Activation Furnace

The activation furnace 2 extends in a flow direction of the carbonized raw material C. The activation furnace 2 has a tubular shape. The activation furnace 2 has an inlet 2A and an outlet 2B. The inlet 2A is located at an upstream end portion of the activation furnace 2 in the flow direction. The outlet 2B is located at a downstream end portion of the activation furnace 2 in the flow direction.

The method for increasing the temperature in the activation furnace 2 is not particularly limited. Examples of method for increasing the temperature in the activation furnace 2 include: heating the activation furnace 2 from outside using an electric heater; combusting a fuel in the activation furnace 2; and combusting a combustible gas such as hydrogen or carbon monoxide generated in the activation reaction. The method for increasing the temperature in the activation furnace 2 can be preferably combusting a fuel in the activation furnace 2. The method for increasing the temperature in the activation furnace 2 can be more preferably combusting a fuel at an inlet 2A side within the activation furnace 2. By combusting a fuel at the inlet 2A side within the activation furnace 2, the temperature of the carbonized raw material supplied through the inlet 2A and the temperature of activation gas supplied by the activation gas supply device 5 can be increased rapidly, thereby rapidly activating the carbonized raw material supplied through the inlet 2A.

The temperature at the inlet 2A side within the activation furnace 2 is, for example, 750°C or more, preferably 850°C or more, more preferably 870°C or more. When the temperature at the inlet 2A side within the activation furnace 2 is equal to or less than the above lower limit, the carbonized raw material can be sufficiently activated in the activation furnace 2. As a result, the packing density of the carbon component of the obtained activated carbon can be adjusted to fall within the above-described range.

The temperature in the activation furnace 2 is, for example, 1200°C or less, preferably 1100°C or less, more preferably 1000°C or less. When the temperature in the activation furnace 2 is maintained at or below the above upper limit, excessive densification of the obtained activated carbon can be suppressed, and the packing density of the carbon component of the obtained activated carbon can be adjusted within the above-described range.

The period of time during which the activation step is performed is not particularly limited as long as the above-described activated carbon can be obtained. The time of the activation step is, for example, 1 hour to 120 hours, preferably 4 hours to 72 hours.

The adsorption performance (e.g., butane activity) of the obtained activated carbon may be measured, and the temperature at the inlet 2A side within the activation furnace 2 may be adjusted so that a desired adsorption performance can be obtained.

Examples of the activation furnace 2 include a rotary kiln and a conveyor kiln. The activation furnace 2 can be preferably the rotary kiln.

### (3-2) Supply Device

The supply device 3 supplies the carbonized raw material C to the inlet 2A of the activation furnace 2. Examples of the supply device 3 include a belt conveyor and a screw feeder.

### (3-3) Conveyance Device

The conveyance device 4 conveys the activated carbon A discharged through the outlet 2B of the activation furnace 2. The conveyance device 4 may be a batch-type device or a continuous-type device. The activated carbon A immediately after being discharged through the outlet 2B is at a high temperature. Therefore, to reduce the risk of combustion of the activated carbon A, the oxygen concentration in the conveyance device 4 is preferably reduced. Examples of the method for reducing the oxygen concentration in the conveyance device 4 include: introducing an inert gas such as nitrogen gas into the conveyance device 4; and introducing exhaust gas from a rotary kiln into the conveyance device 4. The conveyance device 4 preferably includes a cooling device for cooling the activated carbon A. The cooling device may be an air-cooled or water-cooled device.

### (3-4) Activation Gas Supply Device

The activation gas supply device 5 introduces activation gas from an inlet 2A side of the activation furnace 2. In other words, the activation gas supply device 5 introduces activation gas through the upstream end portion of the activation furnace 2 in the flow direction.

Due to this, the concentration of the activation gas in the activation furnace 2 is highest at the inlet 2A side of the activation furnace 2 and decreases toward the outlet 2B of the activation furnace 2. Due to the above reason, the reaction rate of the carbonized raw material C with the activation gas is highest at the inlet 2A side of the activation furnace 2 and decreases toward the outlet 2B of the activation furnace 2.

Thus, the carbonized raw material C supplied into the activation furnace 2 reacts with the high-concentration activation gas, so that pores having small pore radii are formed on the surface of the carbonized raw material C. Thereafter, as the carbonized raw material C moves toward the outlet 2B in the activation furnace 2, the concentration of the activation gas decreases, and the reaction rate of the carbonized raw material C with the activation gas also decreases. This can reduce pore expansion on the surface of the carbonized raw material C due to further progress of the activation reaction. As a result, a decrease in the number of pores having pore radii of 0.6 nm or less and a decrease in the ratio (V_{0.4}/V_{0.5}) can be mitigated.

Examples of the activation gas include water vapor and carbon dioxide. The activation gas can be preferably water vapor. Using water vapor as the activation gas can facilitate an increase in the "pore volume of pores having pore radii of 0.6 nm or less." Accordingly, activated carbon A having excellent anesthetic gas adsorption performance can be obtained. An inert gas such as nitrogen gas or air may be used in combination with the activation gas.

The concentration of the activation gas is not limited as long as the activated carbon as described above can be obtained. The concentration of the activation gas at the inlet 2A side is, for example, 10 vol% to 100 vol%, preferably 20 vol% to 80 vol%.

The activation step may be performed under reduced pressure and may be performed under a nitrogen atmosphere.

The obtained activated carbon may be subjected to crushing, pulverizing, and/or classification. The obtained activated carbon may be washed with water, an organic solvent, an acidic aqueous solution, or an alkaline aqueous solution. The obtained activated carbon may be subjected to an additional heat treatment.

### 3. Anesthetic Gas Adsorption Filter

The anesthetic gas adsorbent described above is suitable for the anesthetic gas adsorption filter 10. The anesthetic gas adsorption filter 10 is used for exhaust gas treatment of the inhalation anesthesia system 100. As shown in FIG. 2, the anesthetic gas adsorption filter 10 includes a container 11 and an anesthetic gas adsorbent 12.

The container 11 contains the anesthetic gas adsorbent 12 described above. The container 11 extends in a first direction. The shape and material of the container 11 are not limited. The container 11 has, for example, a cylindrical shape. The container 11 is made of, for example, hard plastic. The container 11 has an inlet port 11A and an outlet port 11B. The inlet port 11A is located at an end portion of the container 11 on a first side in the first direction. The shape of the inlet port 11A is not particularly limited. The inlet port 11A has, for example, a circular shape. The inlet port 11A has dimensions smaller than the internal dimensions of the container 11. To be more specific, a diameter R1 of the inlet port 11A is smaller than an inner diameter R2 of the container 11. The outlet port 11B is located at another end portion of the container 11 on a second side in the first direction. The location, number, and shape of the outlet port 11B are not limited. One outlet port 11B or a plurality of outlet ports 11B may be provided. Exhaust gas G1 containing anesthetic gas flows into the inlet port 11A. The exhaust gas G1 entering the container 11 through the inlet port 11A passes through the anesthetic gas adsorbent 12. At this time, the anesthetic gas contained in the exhaust gas G1 is adsorbed by the activated carbon of the anesthetic gas adsorbent 12 and is removed from the exhaust gas G1. In other words, the anesthetic gas adsorption filter 10 removes the anesthetic gas from the exhaust gas G1 by allowing the exhaust gas G1 containing the anesthetic gas to pass through the anesthetic gas adsorbent 12. Exhaust gas G2, obtained after the removal of the anesthetic gas, flows out through the outlet port 11B.

The container 11 may include: a container body 111 having an opening 111A; and a lid 112 closing the opening 111A. The container body 111 extends in the first direction. The container body 111 has a cylindrical shape. The opening 111A is located at an end portion of the container body 111 on the first side in the first direction. The diameter of the opening 111A is equal to the inner diameter R2 of the container 11. In assembling the anesthetic gas adsorption filter 10, the anesthetic gas adsorbent 12 is disposed in the container body 111 through the opening 111A. The container body 111 may have the outlet port 11B. The lid 112 may have the inlet port 11A. The lid 112 may be removable from the container body 111.

The anesthetic gas adsorbent 12 is disposed in the container 11. The anesthetic gas adsorbent 12 is loaded into the container 11. The anesthetic gas adsorbent 12 is disposed between the inlet port 11A and the outlet port 11B in the container 11. The anesthetic gas adsorbent 12 is disposed between the inlet port 11A and the outlet port 11B in the first direction.

### 4. Inhalation Anesthesia System

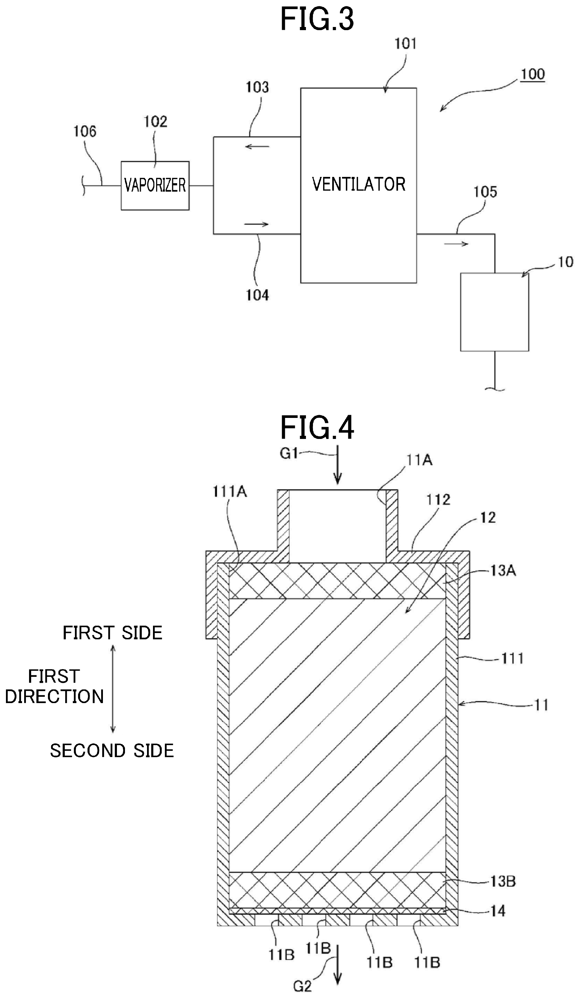

As shown in FIG. 3, the inhalation anesthesia system 100 includes a vaporizer 102 and the anesthetic gas adsorption filter 10 described above. The inhalation anesthesia system 100 may further include a ventilator 101.

The vaporizer 102 is coupled to an inspiratory line 103 of the ventilator 101 and an expiratory line 104 of the ventilator 101. The vaporizer 102 vaporizes a volatile anesthetic agent supplied from an anesthetic agent supply line (not shown) to generate anesthetic gas. The vaporizer 102 supplies the generated anesthetic gas to inspiratory gas from the ventilator 101. The inspiratory gas containing the anesthetic gas is supplied to a subject through a flexible tube 106.

The anesthetic gas adsorption filter 10 is connected to an exhaust line 105 of the ventilator 101. The exhaust gas G1 (see FIG. 2) from the ventilator 101 contains the anesthetic gas contained in exhaled gas from the subject. As described above, the anesthetic gas adsorption filter 10 removes the anesthetic gas from the exhaust gas G1.

### 5. Functions and Effects

(1) The anesthetic gas adsorbent described above includes the activated carbon in which: the "pore volume of pores having pore radii of 0.6 nm or less" is 0.41 mL/g or more; and the ratio (V_{0.4}/V_{0.5}) of the "pore volume of pores having pore radii of 0.4 nm or less" to the "pore volume of pores having pore radii of 0.5 nm or less" is 50% or more.

It is therefore possible to improve the adsorption performance for anesthetic gas contained in exhaust gas.

(2) According to the method for producing the anesthetic gas adsorbent described above, as shown in FIG. 1, in the activation step, the activated carbon A is produced in a continuous manner (i.e., while the carbonized raw material C is continuously supplied to the activation furnace 2, the activated carbon A activated in the activation furnace 2 is continuously discharged from the activation furnace 2). The concentration of the activation gas in the activation furnace 2 is highest at the inlet 2A side of the activation furnace 2 and decreases toward the outlet 2B of the activation furnace 2.

Thus, in the vicinity of the inlet 2A, the carbonized raw material C supplied into the activation furnace 2 reacts with the high-concentration activation gas, so that pores having small pore radii are formed on the surface of the carbonized raw material C.

As the carbonized raw material C thereafter moves toward the outlet 2B in the activation furnace 2, the concentration of the activation gas decreases.

This can reduce pore expansion on the surface of the carbonized raw material C due to further progress of the activation reaction.

As a result, a decrease in the number of pores having pore radii of 0.6 nm or less and a decrease in the ratio (V_{0.4}/V_{0.5}) can be mitigated, and the anesthetic gas adsorbent described above can be efficiently produced.

(3) As shown in FIG. 2, the anesthetic gas adsorption filter 10 includes the anesthetic gas adsorbent 12 described above.

Therefore, the anesthetic gas in the exhaust gas G1 can be efficiently removed.

(4) As shown in FIG. 3, the inhalation anesthesia system 100 includes the anesthetic gas adsorption filter 10 described above. Therefore, the anesthetic gas in the exhaust gas G1 can be efficiently removed.

### 6. Variations

The following describes variations. In the variations, the same reference numerals are given to members similar to those in the above-described embodiment, and descriptions thereof are omitted.
(1) The anesthetic gas adsorbent may further include a bag that contains the activated carbon. The bag is made of, for example, gas-permeable nonwoven fabric.
(2) As shown in FIG. 4, the anesthetic gas adsorption filter 10 may further include cushioning members 13A and 13B and a dust filter 14. In this case, the anesthetic gas adsorbent 12 is loaded into a space between the cushioning member 13A and the cushioning member 13B in the first direction. In assembling the anesthetic gas adsorption filter 10, the dust filter 14, the cushioning member 13B, the anesthetic gas adsorbent 12, and the cushioning member 13A are arranged in this order in the container body 111 through the opening 111A.

The cushioning member 13A is disposed between the inlet port 11A and the anesthetic gas adsorbent 12 in the direction in which the container 11 extends (i.e., the first direction). The cushioning member 13A is disposed between the lid 112 and the anesthetic gas adsorbent 12 in the direction in which the container 11 extends (i.e., the first direction). The cushioning member 13A is made of, for example, gas-permeable nonwoven fabric or gas-permeable polyurethane foam. The cushioning member 13A is in contact with the anesthetic gas adsorbent 12. The cushioning member 13A reduces movement of the anesthetic gas adsorbent 12 within the container 11. The cushioning member 13A also reduces shifting of the anesthetic gas adsorbent 12 within the container 11. Furthermore, the cushioning member 13A reduces spillage of the anesthetic gas adsorbent 12 through the inlet port 11A.

The thickness of the cushioning member 13A is, for example, 5 mm to 30 mm, preferably 10 mm to 25 mm.

The cushioning member 13B is disposed between the outlet port 11B and the anesthetic gas adsorbent 12 in the direction in which the container 11 extends (i.e., the first direction). The cushioning member 13B is made of, for example, gas-permeable nonwoven fabric or gas-permeable polyurethane foam. The cushioning member 13B is in contact with the anesthetic gas adsorbent 12. The cushioning member 13B, together with the cushioning member 13A, reduces movement of the anesthetic gas adsorbent 12 within the container 11. The cushioning member 13B, together with the cushioning member 13A, reduces shifting of the anesthetic gas adsorbent 12 within the container 11. Furthermore, the cushioning member 13A reduces spillage of the anesthetic gas adsorbent 12 through the outlet port 11B.

The thickness of the cushioning member 13B is, for example, 5 mm to 30 mm, preferably 10 mm to 25 mm.

The dust filter 14 is disposed between the outlet port 11B and the cushioning member 13B. The dust filter 14 is made of, for example, gas-permeable nonwoven fabric. The dust filter 14 collects fine powder of the activated carbon passing through the cushioning member 13B. The mesh count of the dust filter 14 is higher than the mesh count of the cushioning members 13A and 13B.

The thickness of the dust filter 14 is less than the thickness of the cushioning member 13B. The thickness of the dust filter 14 is, for example, 0.1 mm to 1.0 mm, preferably 0.2 mm to 0.5 mm.

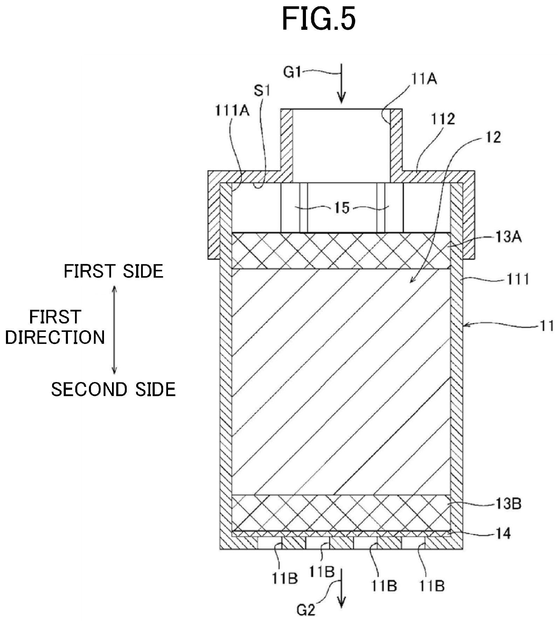

(3) As shown in FIG. 5, the anesthetic gas adsorption filter 10 may further include a spacer 15. The anesthetic gas adsorption filter 10 may include a plurality of spacers 15.

The one or more spacers 15 are disposed between the lid 112 and the cushioning member 13A in the direction in which the container 11 extends (i.e., the first direction). The one or more spacers 15 are disposed between the inlet port 11A and the anesthetic gas adsorbent 12 in the first direction. The one or more spacers 15 are in contact with the cushioning member 13A. The one or more spacers 15 keep the cushioning member 13A spaced apart from the inlet port 11A to secure a space between the inlet port 11A and the cushioning member 13A. Thus, the one or more spacers 15 secure a space between the inlet port 11A and the anesthetic gas adsorbent 12.

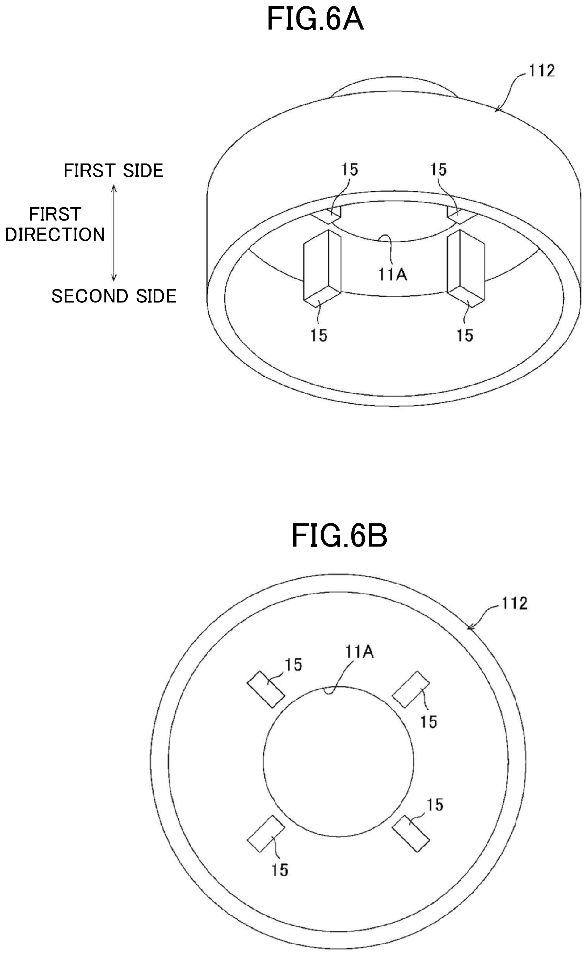

As shown in FIG. 6A, the spacers 15 protrude toward the cushioning member 13A from an inner surface S1 of the lid 112 in the first direction. In other words, the spacers 15 protrude toward the anesthetic gas adsorbent 12 from the inner surface S1 of the lid 112 in the first direction. In other words, the spacers 15 protrude toward the anesthetic gas adsorbent 12 from the inner surface S1 of the container 11 on the first side in the first direction. Each of the spacers 15 is a protrusion. As shown in FIG. 6B, the spacers 15 are arranged around the inlet port 11A. The plurality of spacers 15 are arranged in the circumferential direction of the inlet port 11A. The plurality of spacers 15 are arranged at intervals from each other in the circumferential direction of the inlet port 11A. The spacers 15 extend in the radial direction of the inlet port 11A. The shape and number of the spacers 15 are not particularly limited.

According to this variation, part of the exhaust gas G1 entering the container 11 through the inlet port 11A can be caused to flow in the radial direction of the container 11 in the space between the inlet port 11A and the cushioning member 13A.

This allows the exhaust gas G1 entering the container 11 through the inlet port 11A to be in contact with the entire anesthetic gas adsorbent 12 in the container 11.

As a result, the anesthetic gas contained in the exhaust gas G1 can be efficiently removed.

Furthermore, according to this variation, the one or more spacers 15 protrude from the inner surface S1 of the lid 112 in the first direction.

Therefore, the spacers 15 can be handled together with the lid 112.

As a result, in a single operation of attaching the lid 112 to the container body 111, the lid 112 can be attached to the container body 111, and the spacers 15 can be disposed in the container 11.

Furthermore, the spacers 15 can also be discarded together with the lid 112.

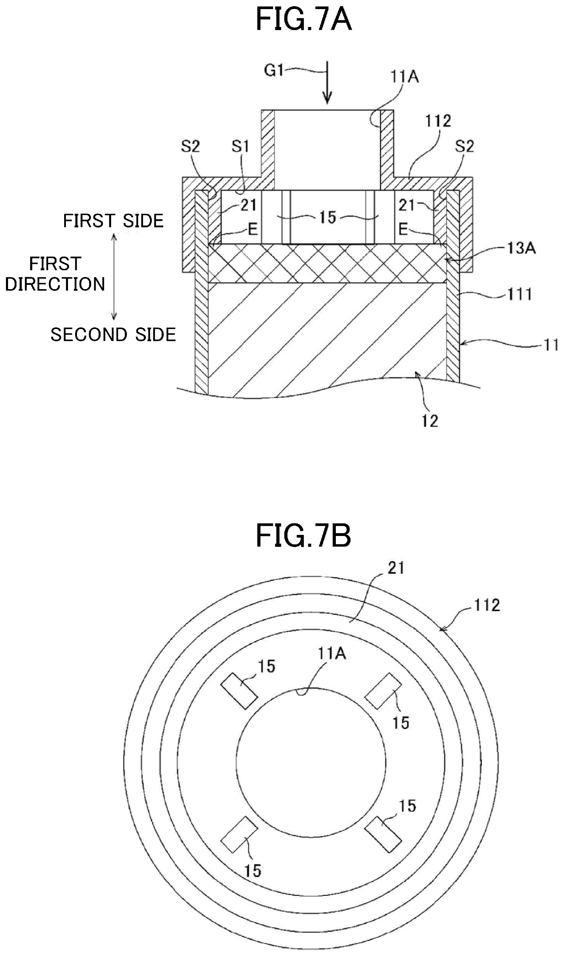

(4) As shown in FIG. 7A, the anesthetic gas adsorption filter 10 may further include a second spacer 21.

The second spacer 21 is disposed between an inner surface S2 of the container 11 and the spacers 15 in a second direction orthogonal to the first direction (specifically, in the radial direction of the inlet port 11A). The second spacer 21 secures, together with the plurality of spacers 15, the space between the inlet port 11A and the anesthetic gas adsorbent 12. Specifically, the second spacer 21 is disposed between the lid 112 and the cushioning member 13A in the first direction. The second spacer 21 is disposed between the inlet port 11A and the anesthetic gas adsorbent 12 in the first direction. The second spacer 21 protrudes toward the cushioning member 13A from the inner surface S1 of the lid 112 in the first direction. The second spacer 21 is in contact with a peripheral edge portion E of the cushioning member 13A. The second spacer 21 presses the peripheral edge portion E of the cushioning member 13A. Thus, the space between the inlet port 11A and the cushioning member 13A can be secured by the second spacer 21 in a region closer to the inner surface S2 of the container 11 than the plurality of spacers 15 in the radial direction of the container 11.

As shown in FIG. 7B, the second spacer 21 has a ring shape. The second spacer 21 extends along the inner surface S2 of the container 11 in the second direction (see FIG. 7A). This can reduce leakage of the exhaust gas G1 in the container 11 through a gap between the container body 111 and the lid 112.

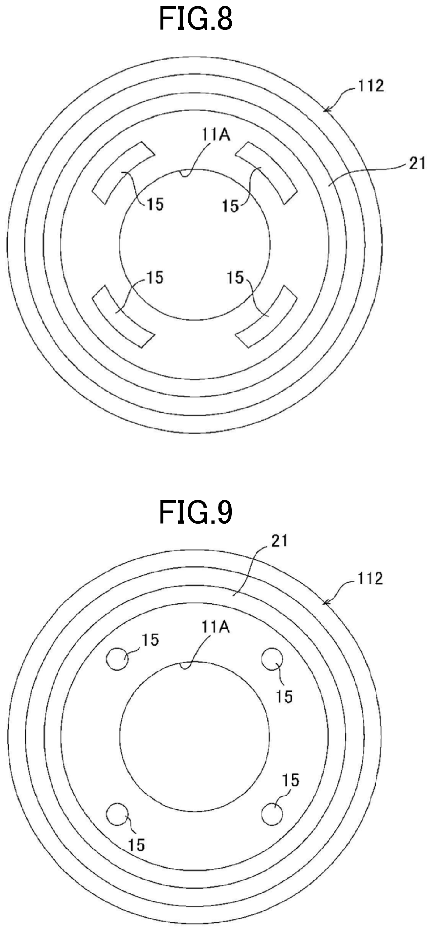

(5) As shown in FIG. 8, each of the plurality of spacers 15 may extend in the circumferential direction of the inlet port 11A.

(6) As shown in FIG. 9, each of the plurality of spacers 15 may have a cylindrical shape.

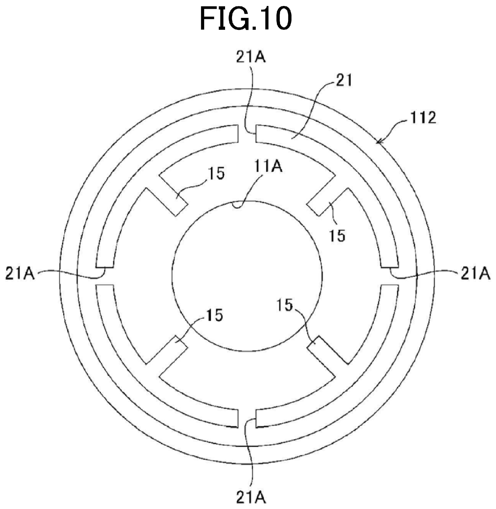

(7) As shown in FIG. 10, the plurality of spacers 15 may be continuous with the second spacer 21. Furthermore, the second spacer 21 may have a plurality of slits 21A. The plurality of slits 21A are arranged at intervals from each other in the circumferential direction of the inlet port 11A.

(8) Variations (1) to (7) can also achieve functions and effects similar to those of the above-described embodiment.

### Examples

Examples are given below to describe the present invention more specifically; however, the present invention is not limited thereto. Specific numerical values such as blending ratios (content ratios), physical property values, and parameters used in the following description can be replaced with upper limits (numerical values defined by "or less," "equal to or less than" or "less than") or lower limits (numerical values defined by "or more," "equal to or greater than," or "more than") of the corresponding numerical values such as blending ratios (content ratios), physical property values, and parameters described in the above "DESCRIPTION OF EMBODIMENTS." Unless otherwise specified, "part(s)" and "%" are on a mass basis.

### 1. Production of Activated Carbon

### (1) Example 1

As the carbonized raw material, carbonized coconut-shell material manufactured in Sri Lanka was prepared.

Subsequently, soil and sand (causing substances causing an ignition residue) were removed from the carbonized raw material using a soil and sand separator (removal step).

Next, the carbonized raw material, from which soil and sand had been removed, was activated using a rotary kiln as the activation furnace. Specifically, while the carbonized raw material, from which soil and sand had been removed, was continuously supplied to the rotary kiln, activated carbon, activated in the rotary kiln, was continuously withdrawn from the rotary kiln.

The inlet temperature of the rotary kiln was adjusted to between 900°C and 1000°C by combusting heavy oil at an inlet side within the rotary kiln.

Furthermore, water vapor serving as the activation gas was introduced through an introduction pipe inserted into an inlet side of the rotary kiln so that the concentration of the activation gas at the inlet side was 20 vol% to 70 vol%. The length of the portion of the introduction pipe that is inserted into the rotary kiln was 2.5% of the total length of the rotary kiln.

Subsequently, the obtained activated carbon was cooled and then crushed to obtain activated carbon serving as the anesthetic gas adsorbent.

### (2) Example 2

Activated carbon was obtained in the same manner as in Example 1 except that the amount of carbonized raw material supplied was changed to 1.1 times that in Example 1.

### (3) Example 3

Activated carbon was obtained in the same manner as in Example 1 except that the amount of carbonized raw material supplied was changed to 1.2 times that in Example 1.

### (4) Example 4

Activated carbon was obtained in the same manner as in Example 1 except that the amount of carbonized raw material supplied was changed to 1.3 times that in Example 1.

### (5) Example 5

Activated carbon was obtained in the same manner as in Example 1 except that deashed coal pellets produced in China were used as the carbonized raw material.

### (6) Example 6

Used coconut-shell-pellet activated carbon (4 × 6 mesh) was prepared as the carbonized raw material.

The carbonized raw material was washed with water to remove soil and sand in the same manner as in Example 1.

Subsequently, activated carbon was obtained in the same manner as in Example 1 except that the fuel was changed to methane.

### (7) Comparative Example 1

Activated carbon was obtained in the same manner as in Example 1 except that the amount of carbonized raw material supplied was changed to 1.5 times that in Example 1.

### (8) Comparative Example 2

Activated carbon was obtained in the same manner as in Example 1 except that deashed coal pellets produced in Colombia were used as the carbonized raw material.

### (9) Comparative Example 3

Activated carbon was obtained in the same manner as in Comparative Example 2 except that the amount of carbonized raw material supplied was changed to 1.1 times that in Comparative Example 2.

### (9) Comparative Example 4

Activated carbon was obtained in the same manner as in Comparative Example 2 except that the amount of carbonized raw material supplied was changed to 1.2 times that in Comparative Example 2.

### 2. Measurement of Physical Properties of Activated Carbon

### (1) Pore Volume and Specific Surface Area

The activated carbon obtained in each of the Examples and Comparative Examples was heated at 150°C for 2 hours under vacuum conditions.

Next, the amount of nitrogen gas adsorbed was measured at -196°C using an automatic specific surface area/pore size distribution analyzer (manufactured by Micromeritics Instrument Corporation (Shimadzu Corporation); model: TRISTAR3000), to prepare a nitrogen adsorption isotherm.

The obtained nitrogen adsorption isotherm was analyzed by the micropore analysis method (MP method), to determine: the pore volume of pores having pore radii of 0.6 nm or less (more specifically, the cumulative pore volume of pores having pore radii of 0.2 nm to 0.6 nm); the pore volume of pores having pore radii of 0.4 nm or less (more specifically, the cumulative pore volume of pores having pore radii of 0.2 nm to 0.4 nm); the pore volume of pores having pore radii of 0.5 nm or less (more specifically, the cumulative pore volume of pores having pore radii of 0.2 nm to 0.5 nm); and the ratio (V_{0.4}/V_{0.5}). The results are shown in Table 1.

Using the nitrogen adsorption isotherm, the specific surface area per 1 g of the activated carbon was calculated by the BET multi-point method from the linear plot in the relative pressure range of 0.01 to 0.10. The results are shown in Table 1.

### (2) Packing Density of Carbon Component

The bulk density of the activated carbon obtained in each of the Examples and Comparative Examples was measured by the method specified in JIS K 1474: 2014.

Specifically, the activated carbon obtained in each of the Examples and Comparative Examples was dried at 115 ± 5°C for 3 hours using a constant-temperature drying oven (DVS402, manufactured by Yamato Scientific Co., Ltd.).

Subsequently, the activated carbon taken out of the constant-temperature drying oven was allowed to cool to room temperature in a desiccator using silica gel as a desiccant.

Thereafter, the cooled activated carbon was introduced into a storage hopper of a bulk density measurement container (TD-V5, manufactured by Toyo Denji Kikai Seisakusho), and using an attached vibrator, the activated carbon was loaded into a 100-mL graduated cylinder up to the 100-mL mark.

The mass of the activated carbon in the graduated cylinder was measured, and the bulk density of the activated carbon was calculated.

The ignition residue of the activated carbon obtained in each of the Examples and Comparative Examples was measured by the method specified in JIS K 1474: 2014.

Specifically, the activated carbon obtained in each of the Examples and Comparative Examples was dried at 115 ± 5°C for 3 hours using a constant-temperature drying oven (DVS402, manufactured by Yamato Scientific Co., Ltd.).

Subsequently, the activated carbon taken out of the constant-temperature drying oven was allowed to cool to room temperature in a desiccator using silica gel as a desiccant.

After the mass of the cooled activated carbon was measured, the activated carbon was subjected to ignition in air at 850 ± 50°C for 1 hour using an electrical furnace (Yamato Scientific Co., Ltd.; model: FO811).

Then, the obtained ash was allowed to cool to room temperature in a desiccator using silica gel as a desiccant, and the mass of ash (i.e., ash mass) was measured, to calculate the ignition residue.

Next, the packing density of the carbon component was calculated by the formula described in the above embodiment. The results are shown in Table 1.

### (3) Butane Activity

The butane activity of the activated carbon obtained in each of the Examples and Comparative Examples was measured by the method specified in ASTM D5742.

Specifically, first, a glass tube specified in ASTM D5742 was dried, and the weight (A) thereof was measured.

Subsequently, the bulk density of the activated carbon, which had been dried in advance by the method specified in ASTM D2867, was measured by the method specified in ASTM D2854.

Next, the mass of activated carbon corresponding to a volume of 16.70 ± 0.05 mL, as calculated from the measured bulk density, was weighed out and loaded into the glass tube, and the total weight (B) was measured.

Then, the glass tube was immersed in a water bath at 25 ± 0.2°C, and butane was passed through the activated carbon in the glass tube at a flow rate of 250 ± 5 mL/min for 900 seconds.

Thereafter, the glass tube was removed from the water bath, wiped clean, and the total weight (C) was measured.

The butane activity was calculated by the following formula. The results are shown in Table 1.

Formula: $Butane activity = \left[\left(C - B\right)/\left(B - A\right)\right] \times 100$

### 3. Evaluation of Activated Carbon Performance

The activated carbon obtained in each of the Examples and Comparative Examples was dried at 115 ± 5°C for 3 hours using the constant-temperature drying oven (DVS402, manufactured by Yamato Scientific Co., Ltd.).

Subsequently, the activated carbon taken out of the constant-temperature drying oven was allowed to cool to room temperature in a desiccator using silica gel as a desiccant.

The mass of activated carbon corresponding to a volume of 300 mL, as calculated from the "bulk density of the activated carbon" described above, was weighed out from the cooled activated carbon and loaded into a test column.

The test column has a structure in which an acrylic resin-made tube having an inner diameter of 50 mm is located between a first lid having an inlet port and a second lid having an outlet port. In the tube, a cushioning member formed of nonwoven fabric, the activated carbon, a cushioning member formed of nonwoven fabric, and a dust filter formed of nonwoven fabric were stacked in this order from the first lid to the second lid. The height of the layer of the activated carbon in the tube (i.e., the size of the layer in the direction in which the tube extends) was approximately 150 mm.

Subsequently, air containing 0.33 mass% of isoflurane at 20 ± 3°C was introduced into the test column packed with the activated carbon through the inlet port at a flow rate of 4.5 L/min.

Thereafter, the cumulative mass of isoflurane introduced into the test column up to the point when the concentration of isoflurane in the air exiting the outlet port exceeded 0.15 mass% was divided by the mass of the packed activated carbon, thereby obtaining the isoflurane removal performance.

**[Table 1]**

| | Pore volume of pores having pore radii of 0.6 nm or less (mL/g) | Ratio (V_{0.4}/V_{0.5}) (%) | Packing density (g/mL) | Ignition residue (mass%) | Packing density of carbon component (g/mL) | Specific surface area (m²/g) | Butane adsorption performance (mass%) | Isoflurane removal performance |
|---|---|---|---|---|---|---|---|---|
| Example 1 | 0.61 | 74.3 | 0.41 | 1.9 | 0.40 | 1503 | 31.8 | 0.68 |
| Example 2 | 0.56 | 84.5 | 0.48 | 2.2 | 0.47 | 1387 | 29.5 | 0.57 |
| Example 3 | 0.52 | 85.6 | 0.49 | 2.4 | 0.47 | 1291 | 27.3 | 0.56 |
| Example 4 | 0.46 | 86.0 | 0.50 | 1.7 | 0.49 | 1135 | 24.1 | 0.54 |
| Example 5 | 0.45 | 56.5 | 0.44 | 11.4 | 0.39 | 1087 | 28.0 | 0.51 |
| Example 6 | 0.48 | 52.9 | 0.42 | 1.7 | 0.42 | 1193 | 25.3 | 0.52 |
| Comparative Example 1 | 0.40 | 91.7 | 0.51 | 1.4 | 0.50 | 996 | 22.0 | 0.43 |
| Comparative Example 2 | 0.46 | 49.7 | 0.48 | 9.4 | 0.44 | 1089 | 28.1 | 0.46 |
| Comparative Example 3 | 0.43 | 55.6 | 0.43 | 10.9 | 0.38 | 1024 | 26.3 | 0.44 |
| Comparative Example 4 | 0.40 | 65.4 | 0.47 | 9.6 | 0.43 | 954 | 24.3 | 0.43 |

Illustrative embodiments of the present invention have been presented above; however, these embodiments are provided merely by way of example, and the present invention should not be construed as being limited thereto. Variations and modifications of the present invention that are apparent to those skilled in the art are intended to be included within the scope of the following claims.

### INDUSTRIAL APPLICABILITY

The anesthetic gas adsorbent, the anesthetic gas adsorption filter, and the method for removing anesthetic gas of the present invention can be used to remove anesthetic gas. The inhalation anesthesia system of the present invention can be used to perform inhalation anesthesia.

### DESCRIPTION OF REFERENCE CHARACTERS

- 10: Anesthetic Gas Adsorption Filter
- 11: Container
- 11A: Inlet Port
- 11B: Outlet Port
- 12: Anesthetic Gas Adsorbent
- 15: Spacer
- G1: Exhaust Gas Containing Anesthetic Gas
- G2: Exhaust Gas after Removal of Anesthetic Gas
- 100: Inhalation Anesthesia System
- 101: Ventilator
- 102: Vaporizer
- 105: Exhaust Line

## Claims

1. An anesthetic gas adsorbent comprising:
activated carbon,
a "pore volume of pores having pore radii of 0.6 nm or less" of the activated carbon calculated by MP method being 0.41 mL/g or more,
a ratio of a "pore volume of pores having pore radii of 0.4 nm or less" of the activated carbon calculated by the MP method to a "pore volume of pores having pore radii of 0.5 nm or less" of the activated carbon calculated by the MP method being 50% or more.

2. The anesthetic gas adsorbent of claim 1, wherein
a packing density of the activated carbon excluding an ignition residue is more than 0.38 g/mL and less than 0.50 g/mL.

3. The anesthetic gas adsorbent of claim 1, wherein
the "pore volume of pores having pore radii of 0.6 nm or less" of the activated carbon calculated by the MP method is 1.70 mL/g or less.

4. The anesthetic gas adsorbent of claim 1, wherein
a specific surface area of the activated carbon is 950 m²/g or more and 2000 m²/g or less, and
a butane activity of the activated carbon is 24.4% or more and 40.0% or less.

5. An anesthetic gas adsorption filter comprising:
the anesthetic gas adsorbent of claim 1.

6. The anesthetic gas adsorption filter of claim 5, further comprising:
a container having
an inlet port that is located at an end portion of the container on a first side in a first direction and that allows exhaust gas containing anesthetic gas to flow into the container, and
an outlet port that is located at another end portion of the container on a second side in the first direction and that allows the exhaust gas, from which the anesthetic gas has been removed, to flow out of the container;
the anesthetic gas adsorbent disposed between the inlet port and the outlet port in the first direction; and
a spacer disposed between the inlet port and the anesthetic gas adsorbent in the first direction.

7. The anesthetic gas adsorption filter of claim 6, wherein
the spacer is a protrusion protruding from an inner surface of the container on the first side in the first direction toward the anesthetic gas adsorbent.

8. An inhalation anesthesia system comprising:
a vaporizer configured to vaporize a volatile anesthetic agent to generate anesthetic gas and to supply the generated anesthetic gas to inspiratory gas from a ventilator; and
an anesthetic gas adsorption filter connected to an exhaust line of the ventilator and including the anesthetic gas adsorbent of claim 1.

9. The inhalation anesthesia system of claim 8, further comprising:
the ventilator.

10. A method for removing anesthetic gas, the method comprising:
passing exhaust gas containing anesthetic gas through the anesthetic gas adsorbent of claim 1, thereby removing the anesthetic gas from the exhaust gas.
